# EUROPÄISCHE PATENTSCHRIFT

(11) **EP 3 914 724 B1**
(45) Veröffentlichungstag und Bekanntmachung des Hinweises auf die Patenterteilung: **25.06.2025**
(21) Anmeldenummer: 20703162.6
(22) Anmeldetag: 24.01.2020
(51) Int. Cl.: C12P 11/00

(54) **VERFAHREN ZUR HERSTELLUNG EINES LIGNOSULFONAT-POLYMERS**
METHOD FOR PRODUCING A LIGNOSULFONATE POLYMER
PROCÉDÉ POUR LA PRÉPARATION D'UN POLYMÈRE DE LIGNOSULFONATE

(30) Priorität: 24.01.2019 AT 500532019
(43) Veröffentlichungstag der Anmeldung: 01.12.2021
(73) Patentinhaber: Universität für Bodenkultur Wien, 1180 WIEN (AT)
(72) Erfinder: NYANHONGO, Gibson Stephen, 3430 Tulln (AT); GUEBITZ, Georg, 3430 Tulln (AT); ORTNER, Andreas, 3430 Tulln (AT); BISCHOF, Sabrina, 3430 Tulln (AT)
(74) Vertreter: Puchberger & Partner Patentanwälte
(86) Internationale Anmeldenummer: PCT/EP2020/051720
(87) Internationale Veröffentlichungsnummer: WO 2020/152314

(56) Entgegenhaltungen:
- DE-A1- 19 700 904
- DE-A1- 19 828 483
- DE-A1- 3 037 992
- DE-C2- 3 037 992
- ARESKOGH DIMITRI ET AL: "Investigation of the Molecular Weight Increase of Commercial Lignosulfonates by Laccase Catalysis", BIOMACROMOLECULES, vol. 11, no. 4, 23 February 2010 (2010-02-23), US, pages 904 - 910, XP055912367, ISSN: 1525-7797, DOI: 10.1021/bm901258v
- DANIELA HUBER ET AL: "Influence of Oxygen and Mediators on Laccase-Catalyzed Polymerization of Lignosulfonate", ACS SUSTAINABLE CHEMISTRY & ENGINEERING, vol. 4, no. 10, 21 June 2016 (2016-06-21), US, pages 5303 - 5310, XP055688738, ISSN: 2168-0485, DOI: 10.1021/acssuschemeng.6b00692
- DANIELA HUBER ET AL: "Polymerization of Various Lignins via Immobilized Myceliophthora thermophila Laccase (MtL)", POLYMERS, vol. 8, no. 8, 3 August 2016 (2016-08-03), pages 280, XP055493944, DOI: 10.3390/polym8080280
- ORTNER ANDREAS ET AL: "Laccase mediated oxidation of industrial lignins: Is oxygen limiting?", PROCESS BIOCHEMISTRY, vol. 50, no. 8, 2015, pages 1277 - 1283, XP029189757, ISSN: 1359-5113, DOI: 10.1016/J.PROCBIO.2015.05.003
- GOUVEIA S ET AL: "Polymerisation of Kraft lignin from black liquors by laccase from Myceliophthora thermophila: Effect of operational conditions and black liquor origin", BIORESOURCE TECHNOLOGY, ELSEVIER, AMSTERDAM, NL, vol. 131, 3 January 2013 (2013-01-03), pages 288 - 294, XP028989235, ISSN: 0960-8524, DOI: 10.1016/J.BIORTECH.2012.12.155

## Beschreibung

Die Erfindung betrifft ein Verfahren zur Herstellung eines Lignosulfonat-Polymers, ein Lignosulfonat-Polymer, hergestellt mit einem erfindungsgemäßen Verfahren, sowie ein Wachstumssubstrat, welches ein erfindungsgemäßes Lignosulfonat-Polymer umfasst.

Lignosulfonate werden auch als Ligninsulfonate bezeichnet und sind Salze der Ligninsulfonsäure. Ligninsulfonsäure ist ein wasserlösliches Polymer, welches im Wesentlichen aus den Monomereinheiten Cumarylalkohol, Coniferylalkohol und Sinapylalkohol besteht, wobei einzelne Hydroxylgruppen (-OH) durch Sulfonylgruppen (-SO₃⁻) substituiert sind.

Lignosulfonate fallen als Nebenprodukt bei der Herstellung von Cellulose nach dem Sulfitverfahren an. Derart hergestellte Lignosulfonatlösungen sind beispielsweise ein Abfallstoff bei der Papierherstellung und werden im Stand der Technik als Rohlauge oder Braunlauge bezeichnet. Neben Lignosulfonaten enthält die Rohlauge zusätzlich üblicherweise Grobbestandteile, wie beispielsweise Ligninfasern oder -partikel, sowie niedermolekulare Bestandteile, wie etwa Salze oder organische Verbindungen.

Da Rohlauge in industriellen Verfahren in großen Mengen als Abfallprodukt anfällt, wäre es wünschenswert, diese als Rohstoff zur Herstellung von bestimmten Produkten zu verwenden. Rohlauge findet beispielsweise Einsatz bei der Zubereitung von Netzmitteln, Dispergiermitteln, Bindemitteln und Kleber Verwendung, wobei große Mengen auch lediglich zur Energiegewinnung durch Verbrennung eingesetzt werden.

Als Lignosulfonat-Polymere werden in Zusammenhang mit der vorliegenden Erfindung derartige Lignosulfonat-Strukturen bezeichnet, bei welchen eine weitere Quervernetzung der nativen Lignosulfonat-Einheiten stattgefunden hat. Bevorzugt sind Lignosulfonat-Polymere im Vergleich zu Lignosulfonaten im Wesentlichen wasserunlöslich.

Im Stand der Technik sind unterschiedliche Lignosulfonat-Polymere bekannt:
So beschreibt beispielsweise der Artikel mit dem Titel "Investigation of the Molecular Weight of Commerical Lignosulfonates by Laccase Catalysis", Biomacromolecules 2010, 11, 904-910, ein Verfahren zur Herstellung eines Lignosulfonat-Polymers, jedoch wird nicht beschrieben, wie wasserunlösliche Polymere gebildet werden können.

DE 3037992 A1 beschreibt ein Verfahren zur Herstellung eines Bindemittels für Spanplatten.

Die DE 19700904 A1 beschreibt ein Zwischenprodukt für die Herstellung von Polymerisaten aus Ligninderivaten. Es wird beschrieben, dass Ligninderivate einer enzymatischen Behandlung unterworfen werden.

Der Artikel mit dem Titel "Polymerization of Various Lignins via Immobilized Myceliophthora thermophila Laccase (MtL)", Polymers 2016, 8, 280 sowie der Artikel mit dem Titel "Laccase mediated oxidation of industrial lignins: Is oxygen limiting?", Process Biochemistry 2015, 50, 1277-1283 beschreiben Lignosulfonat-Salze als Edukte für Polymerisationsreaktionen.

Der Artikel mit dem Titel "Polymerisation of Kraft lignin from black liquors by laccase from Myceliophthora thermophila: Effect of operational conditions and black liquor origin", Biosource Technology 2013, 131, 288-294 beschreibt nicht die Polymerisierung von Lignosulfonat, sondern von Kraftlignin aus Schwarzlauge.

Mit im Stand der Technik bekannten Verfahren ist es bislang nicht möglich, Lignosulfonat-Polymere herzustellen, die im Wesentlichen vollständig auf Lignosulfonatbasis gebildet sind. Es ist gemäß dem Stand der Technik notwendig, die Lignosulfonat-Einheiten mit Quervernetzern zu vernetzen und dadurch Copolymere zu bilden. So können Lignosulfonat-Polymere mit einem hinreichend hohen durchschnittlichen Molekulargewicht gebildet werden, um die gewünschten chemischen und physikalischen Eigenschaften zu zeigen. Eine derartige Copolymerisation erfordert den Einsatz zusätzlicher Rohstoffe und ist im Sinne der Wirtschaftlichkeit eines Herstellungsverfahrens für Lignosulfonat-Polymere unerwünscht.

Die Aufgabe der vorliegenden Erfindung ist es, die Probleme des Standes der Technik zu überwinden und ein Verfahren zu schaffen, mit welchem es möglich ist, wasserunlösliche Lignosulfonat-Polymere zu schaffen, die im Wesentlichen vollständig auf Lignosulfonat-Basis aufgebaut sind und die insbesondere ohne die Verwendung von quervernetzenden Zusatzstoffen auskommen.

Die Aufgabe der Erfindung wird durch die Merkmale der Patentansprüche gelöst. Bevorzugte Merkmale ergeben sich aus den abhängigen Patentansprüchen.

Die Bezeichnung "wasserunlöslich" bezeichnet in Zusammenhang mit der vorliegenden Erfindung, dass ein Stoff, insbesondere ein Lignosulfonat-Polymer, im Wesentlichen nicht in Wasser löslich ist. Insbesondere kann "wasserunlöslich" oder "im Wesentlichen wasserunlöslich" bedeuten, dass weniger als 0,1 Gew.-% der Gesamtmasse eines betreffenden Stoffs bei einer Temperatur von 20°C in Wasser löslich sind.

Die Erfindung betrifft ein Verfahren zur Herstellung eines wasserunlöslichen Lignosulfonat-Polymers aus einer Lignosulfonat-Precursor enthaltenden Lösung, nämlich aus Rohlauge, umfassend die Schritte gemäß Anspruch 1.

In Zusammenhang mit der vorliegenden Erfindung bezeichnet der Begriff "Lignosulfonat-Precursor" eine Mischung aus wasserlöslichen Lignosulfonat-Oligomeren. Die Ligninsulfonat-Oligomere können insbesondere aus den Teilstrukturen bzw. aus den Monomereinheiten Cumarylalkohol, Coniferylalkohol und Sinapylalkohol aufgebaut sein, wobei einzelne Hydroxylgruppen durch Sulfonylgruppen substituiert sind. Eine wichtige, chemisch strukturelle Eigenschaft der Lignosulfonat-Precursor gemäß der vorliegenden Erfindung ist das Vorhandensein von reaktionsfähigen phenolischen OH-Gruppen, welche durch die erfindungsgemäßen radikalbildenden Enzyme bzw. das radikalbildende Enzym zu einem Sauerstoffradikal reduzierbar sind.

Der Begriff "radikalbildendes Enzym" kann in Zusammenhang mit der vorliegenden Erfindung jegliches Enzym bezeichnen, das aus Laccasen und/oder Peroxidasen ausgewählt ist. Bevorzugt werden die radikalbildenden Enzyme aus einer mikrobiellen Kultur, insbesondere aus einer Pilzkultur, gewonnen. Gegebenenfalls kann vorgesehen sein, dass eines oder mehrere radikalbildende Enzyme zur Bildung der Lignosulfonat-Radikale vorgesehen sind.

Das im zweiten Schritt des erfindungsgemäßen Verfahrens vorgesehene Begasen der Lignosulfonat-Precursor-Lösung mit Sauerstoff bezeichnet im Wesentlichen jedes im Stand der Technik bekannte Verfahren zur Versorgung einer Lösung mit einem Gas. Geeignete Verfahren sind einem Fachmann hinlänglich bekannt und umfassen beispielsweise das Einblasen von Sauerstoff durch eine Sinterfritte. Gegebenenfalls kann die Verfügbarkeit von Sauerstoff in der Reaktionsmischung durch starkes Rühren verbessert werden.

Gegebenenfalls ist vorgesehen, dass die Lignosulfonat-Precursor wasserlösliche Lignosulfonat-Oligomere mit einem Molekulargewicht von über 500 Da, bevorzugt mit einem Molekulargewicht von über 5000 Da sind. Es kann also vorgesehen sein, dass die Lignosulfonat-Precursor keine auf Lignosulfonaten basierenden Moleküle enthalten, die ein Molekulargewicht von weniger als 500 Da, bevorzugt von weniger als 5000 Da aufweisen.

Gegebenenfalls ist vorgesehen, dass die Lösung eine wässrige Lösung ist.

Es ist vorgesehen, dass das radikalbildende Enzym eine Laccase, eine Peroxidase oder eine Mischung aus mehreren dieser Enzyme ist. Gegebenenfalls können Mischungen unterschiedlicher Laccasen oder Peroxidasen verwendet werden.

Es ist vorgesehen, dass Sauerstoff als Gas mit einer Beimischung von höchstens 10 Vol.-%, vorzugsweise höchstens 5 Vol.-%, an anderen Gasen zugegeben wird. Dies bedeutet beispielsweise, dass der zugegebene Sauerstoff eine Zumischung von etwa 2% Stickstoff oder anderen Gasen enthalten kann. Bevorzugt ist jedoch, dass Sauerstoff in Form von reinem Sauerstoff ohne wesentliche Zumischung von anderen Gasen verwendet wird. Übliche Verunreinigungen, die in gasförmigem Sauerstoff enthalten sind, können hierbei in geringen Mengen enthalten sein.

Der erste Verfahrensschritt, nämlich der Schritt des Abtrennens oder Selektierens der Lignosulfonat-Precursor, umfasst die folgenden Schritte:
- Filtrieren von Bestandteilen mit einem Partikeldurchmesser von kleiner als 1 µm, insbesondere von kleiner als 100 nm,
   und/oder
- Abscheiden von niedermolekularen Bestandteilen, insbesondere von Salzen und niedermolekularen organischen Verbindungen, bevorzugt von Bestandteilen mit Molekulargewicht von kleiner als 500 Da, bevorzugt von kleiner als 5000 Da.

Dadurch wird ermöglicht, die eine für die Polymerisation der Lignosulfonat-Precursor bevorzugte Molekulargewichtsfraktion zu erhalten. Einerseits kann durch die optional vorgesehenen Verfahrensschritte eine Entfernung von Grobpartikeln oder -fasern erreicht werden, andererseits kann es ermöglicht werden, bei der Polymerisation gegebenenfalls störende niedermolekulare Bestandteile aus der Lignosulfonat-Precursor-Fraktion zu entfernen.

Gegebenenfalls kann vorgesehen sein, dass der pH-Wert der Lösung im zweiten Verfahrensschritt zwischen 5 und 8, bevorzugt zwischen 6,5 und 7,5 liegt. Bevorzugt wird der pH-Wert der Lösung an den optimalen Aktivitätsbereich des jeweils verwendeten radikalbildenden Enzyms angepasst. Die Einstellung des pH-Werts kann mit einem beliebigen im Stand der Technik bekannten Verfahren erfolgen, insbesondere durch die Zugabe eines basischen bzw. eines sauren Stoffes. Ein basischer Stoff kann beispielsweise Natronlauge (NaOH) sein. Ein saurer Stoff kann beispielsweise Chlorwasserstoffsäure (HCl) sein.

Gegebenenfalls ist vorgesehen, dass während oder nach der Polymerisation wenigstens ein Zusatzstoff zugegeben wird, wobei der Zusatzstoff vorzugsweise ausgewählt ist aus Elastomeren, Weichmachern, Stabilisatoren und Polymeren. Bevorzugt erfolgt durch Zugabe des Zusatzstoffs keine Copolymerisation von Lignosulfonat-Einheiten mit dem Zusatzstoff, es kommt also im Wesentlichen zu keiner kovalenten Bindung zwischen Molekülen des Zusatzstoffs oder der Zusatzstoffe und dem Lignosulfonat-Polymer.

Gegebenenfalls kann die Zugabe von Zusatzstoffen die Eigenschaften des erhaltenen Lignosulfonat-Polymers verbessern. Beispielsweise kann durch Zugabe von Weichmachern, wie etwa Sorbitol, Xylitol oder Glycerin, die Zugfestigkeit des Lignosulfonat-Polymers verbessert werden. Als Weichmacher kann jeder Stoff enthalten sein, der einem Fachmann als Weichmacher bekannt ist. Weichmacher können mit einem Anteil von 5 Gew.-% bis 50 Gew.-% im Polymer enthalten sein.

Gegebenenfalls umfasst das erfindungsgemäße Verfahren zusätzlich den Schritt des Aufschäumens der Lignosulfonat-Precursor-Lösung während der Polymerisation zur Bildung eines Lignosulfonat-Polymer-Schaumes. Zur Bildung eines Lignosulfonat-Polymer-Schaumes kann beispielsweise Natriumcarbonat oder Natriumhydrogencarbonat in Verbindung mit einer Säure zu der Reaktionsmischung zugegeben werden. Während der Bildung von Kohlendioxid durch die Reaktion des Carbonats / Hydrogencarbonats mit der Säure erfolgt die Polymerisation der Lignosulfonat-Precursor und es entsteht ein fester bzw. gelartiger Lignosulfonat-Polymer-Schaum. Derartige Lignosulfonat-Polymer-Schäume können in besonders vorteilhafter Weise zur Speicherung von Wasser eingesetzt werden.

Beschrieben wird auch ein Lignosulfonat-Polymer-Schaum, welcher mit einem erfindungsgemäßen Verfahren hergestellt ist. Ein derartiger Lignosulfonat-Polymer-Schaum kann gegebenenfalls eine Porosität zwischen 20 % und 90 % aufweisen. Dabei bezeichnet die Porosität das Verhältnis zwischen Hohlraumvolumen und Gesamtvolumen.

Gegebenenfalls ist vorgesehen, dass das Verfahren zusätzlich den Schritt des Gießens der Lignosulfonat-Precursor-Lösung während der Polymerisation umfasst, um ein Lignosulfonat-Polymer-Material zu bilden. Insbesondere kann dazu vorgesehen sein, dass die Reaktionsmischung noch im flüssigen Zustand in zu einer Schicht mit einer im Wesentlichen konstanten Dicke vergossen wird, woraufhin dann die vollständige Polymerisation erfolgt. Nach dem Trocknen des Polymers wird ein Lignosulfonat-Polymer-Material, insbesondere ein Biokunststoff, erhalten. Bevorzugt erfolgt vor der vollständigen Polymerisation die Zugabe eines Weichmachers, wie beispielsweise Sorbitol oder Glycerin, um die Zugfestigkeit des erhaltenen Polymermaterials zu erhöhen.

Beschrieben wird ferner ein Biokunststoff aus Lignosulfonat-Polymer-Material, welcher mit einem erfindungsgemäßen Verfahren hergestellt ist.

Um einen Biokunststoff aus Lignosulfonat-Polymer-Material herzustellen, kann vorgesehen sein, dass die in einem erfindungsgemäßen Verfahren hergestellte Reaktionslösung ausgegossen wird. Dies bedeutet, dass die noch flüssige Reaktionslösung in eine beliebige Form gegossen wird. Derartige Formen können beispielsweise Folien, Filme, Schäume oder massive Körper unterschiedlicher Geometrie sein.

Die Dicke eines Films oder einer Folie kann zwischen 10 µm und 500 µm, bevorzugt zwischen 50 µm und 500 µm betragen. Der aus einem erfindungsgemäßen Verfahren hergestellte Biokunststoff kann jedoch auch größere Dicken aufweisen, um einen massiven Körper zu bilden.

Bevorzugt umfasst ein erfindungsgemäßes Material zusätzlich einen Weichmacher, wie beispielsweise Sorbitol oder Glycerin, um die Elastizität und die Reißfähigkeit zu verbessern.

Gegebenenfalls ist vorgesehen, dass die Aktivität des radikalbildenen Enzyms nach dessen Zugabe zu der Reaktionsmischung zwischen 10 nkat/mL und 500 nkat/mL, insbesondere zwischen 50 nkat/mL und 200 nkat/mL beträgt. Die Einheit "nkat/mL" ist eine im Bereich der enzymatischen Katalyse gebräuchliche Einheit für die katalytische Aktivität. 1 kat ist jene Menge eines Enzyms, die 1 mol Substrat in 1 Sekunde umsetzt.

Beschrieben wird weiterhin ein Lignosulfonat-Polymer, welches durch ein erfindungsgemäßes Verfahren hergestellt wurde. Bevorzugt ist das mit dem erfindungsgemäßen Verfahren hergestellte Lignosulfonat-Polymer bei Raumtemperatur, also insbesondere bei 25°C, ein Feststoff. Die Polymerisation wird im erfindungsgemäßen Verfahren also bevorzugt so lange durchgeführt, bis ein Feststoff erhalten wird. Gegebenenfalls hat das so erhaltene Polymer ein mittleres Molekulargewicht von 200 kDa bis 800 kDa, insbesondere von 300 kDa bis 500 kDa.

Beschrieben wird auch ein Wachstumssubstrat für Pflanzen, welches zwischen 0,1 Gew.-% und 5 Gew.-%, vorzugsweise zwischen 0,5 Gew.-% und 2 Gew.-% eines erfindungsgemäßen Lignosulfonat-Polymers umfasst. Die Mengenangabe bezieht sich auf trockenes Lignosulfonat-Polymer. Gegebenenfalls kann das Wachstumssubstrat zwischen 1 Gew.-% und 40 Gew.-% eines Hydrogels, also eines vollständig in Wasser aufgequollenen Lignosulfonat-Polymers enthalten.

Durch die gute wasserspeichernde Wirkung der durch das erfindungsgemäße Verfahren erhaltenen Lignosulfonat-Polymere kann das Lignosulfonat in bevorzugter Weise in einem Wachstumssubstrat zur Wasserspeicherung eingesetzt werden. Ein derartiges Wachstumssubstrat kann neben dem Lignosulfonat-Polymer Erde, Sand, Lehm oder andere Bestandteile umfassen, die typischerweise in einem Wachstumssubstrat für Pflanzen enthalten sind.

Das Lignosulfonat-Polymer kann im Wachstumssubstrat insbesondere in Form eines Lignosulfonat-Hydrogels, enthalten sein. Ein Hydrogel bezeichnet im Zusammenhang mit der vorliegenden Erfindung insbesondere ein Polymer, welches einen hohen Wasseranteil aufweist bzw. mit Wasser gesättigt ist. Ein derartiges Hydrogel kann einen Wasseranteil von wenigstens 50%, vorzugsweise von wenigstens 80% oder höher, aufweisen.

Beschrieben wird auch ein Wasserspeichermittel für ein Wachstumssubstrat, wobei das Wasserspeichermittel ein erfindungsgemäßes Lignosulfonat-Polymer umfasst oder daraus besteht.

Das Lignosulfonat-Polymer, insbesondere das Wasserspeichermittel, kann gegebenenfalls in Form eines wasserhaltigen Hydrogels oder in trockener Form vorliegen. In trockener Form kann das Polymer in Form eines Granulats, eines Pulvers oder in bekannten anderen Formen vorliegen.

Die Wasseraufnahmekapazität des erfindungsgemäßen Lignosulfonat-Polymers beträgt gegebenenfalls bis zu 2000% in Bezug auf das Trockengewicht des Polymers.

Das Lignosulfonat-Polymer kann biologisch abbaubar sein. Dadurch dient es bei der Verwendung als Wasserspeichermittel für ein Wachstumssubstrat zusätzlich selbst als Düngemittel. Trotz der biologischen Abbaubarkeit kann die Fähigkeit der Wasserspeicherung in natürlichem Umfeld, also beispielsweise in Erde, für mehrere Jahre, beispielsweise zwei bis fünf Jahre, aufrecht bleiben. Damit steht das erfindungsgemäße Lignosulfonat-Polymer bei Verwendung als Wasserspeichermittel im Gegensatz zu konventionellen Wasserspeichermitteln, die weder biologisch abbaubar sind noch einen inhärenten Düngeeffekt aufweisen.

Gegebenenfalls kann gemäß nicht anspruchsgemäßen Ausführungen in allen Ausführungsformen des Verfahrens das radikalbildende Enzym durch ein anderes geeignetes radikalbildendes Mittel ersetzt werden. Ein derartiges radikalbildendes Mittel kann beispielsweise ein konventioneller chemischer Radikalstarter sein.

Das Lignosulfonat-Polymer kann gegebenenfalls nicht nur Wasser, sondern auch in Wasser gelöste Substanzen speichern. Derartige Substanzen können Salze, Düngemittel, Pflanzenschutzmittel, andere Agrochemikalien und dergleichen sein. Das erfindungsgemäße Lignosulfonat-Polymer kann daher zur kontinuierlichen Abgabe von Wasser und weiteren Substanzen geeignet sein. Dadurch kann beispielsweise ein Auswaschen von Agrochemikalien aus dem Boden verringert werden.

Weitere Merkmale der Erfindung ergeben sich aus den Patentansprüchen, den Figuren, sowie aus den Ausführungsbeispielen.

Im Folgenden wird die Erfindung anhand von exemplarischen Ausführungsbeispielen im Detail erläutert. Die Ausführungsbeispiele sollen lediglich der Illustration der Erfindung dienen und den Schutzumfang nicht beschränken.

Die angefügten Abbildungen dienen der weiteren Verdeutlichung der Wirkungen der vorliegenden Erfindung. Es zeigen:
Fig. 1 den Verlauf der Viskosität in Abhängigkeit von der Reaktionsdauer in einem ersten erfindungsgemäßen Verfahren;
Fig. 2 den Verlauf des durchschnittlichen Molekulargewichts in Abhängigkeit von der Reaktionsdauer im ersten erfindungsgemäßen Verfahren;
Fig. 3 die Wasseraufnahme eines nach dem erfindungsgemäßen Verfahren hergestellten und getrockneten Lignosulfonat-Polymers;
Fig. 4 den Verlauf der relativen Feuchtigkeit von Wachstumssubstraten umfassend ein erfindungsgemäßes Lignosulfonat-Polymer;
Fig. 5 die Zugfestigkeit von erfindungsgemäßen Lignosulfonat-Polymer-Materialien;
Fig. 6 die Reißdehnung von erfindungsgemäßen Lignosulfonat-Polymer-Materialien.

### Beispiel 1 - Herstellung eines Lignosulfonat-Polymers

Das erste Ausführungsbeispiel beschreibt die Herstellung eines wasserunlöslichen Lignosulfonat-Polymers, insbesondere eines Hydrogels, welches ein Lignosulfonat-Polymer enthält, nach einem erfindungsgemäßen Verfahren.

Ausgangsstoff für das erfindungsgemäße Verfahren gemäß dem ersten Ausführungsbeispiel ist Rohlauge, wie sie üblicherweise in der als Abfallprodukt anfällt.

Im ersten Schritt des erfindungsgemäßen Verfahrens wird die Rohlauge filtriert, um Feststoffe, wie beispielsweise Fasern oder Partikel zu entfernen. Es wird ein Filter mit einer Maschenweite von etwa 5 µm verwendet.

Nachfolgend wird ein Ultrafiltrationsschritt durchgeführt, um Salze, niedermolekulares Lignin und andere Verunreinigungen der Rohlauge zu entfernen, die einen negativen Einfluss auf die Polymerisation oder auf die Qualität des Polymerisats haben könnten. Ultrafiltrationsschritte werden mit Membranen unterschiedlicher Retentionseigenschaften durchgeführt, nämlich 20 kDa, 5 nm und 10 nm. Dadurch wird eine hinreichend reine Lignosulfonat-Precursor-Lösung erhalten. Zusätzlich erfolgt eine Aufkonzentrierung der Lignosulfonat-Precursor in der wässrigen Lösung. In dem hier beschriebenen Ausführungsbeispiel beträgt die Konzentration der Lignosulfonat-Precursor nach allen Filtrationsschritten etwa 11 Gew.-%. Das durchschnittliche Molekulargewicht der Lignosulfonat-Precursor in der aufbereiteten Lösung beträgt etwa 26 kDa.

2 L der wie oben vorbereiteten Lösung werden unter Zugabe von 1 M NaOH-Lösung auf einen pH-Wert von etwa 7,0 eingestellt. Laccase aus *Myceliophthora thermophila* wird bis auf eine finale Enzymaktivität von etwa 160 nkat/mL zugegeben. Die Reaktionstemperatur beträgt in diesem Ausführungsbeispiel etwa 40 °C. Die Reaktionstemperatur kann bevorzugt zwischen 20°C und 70°C liegen.

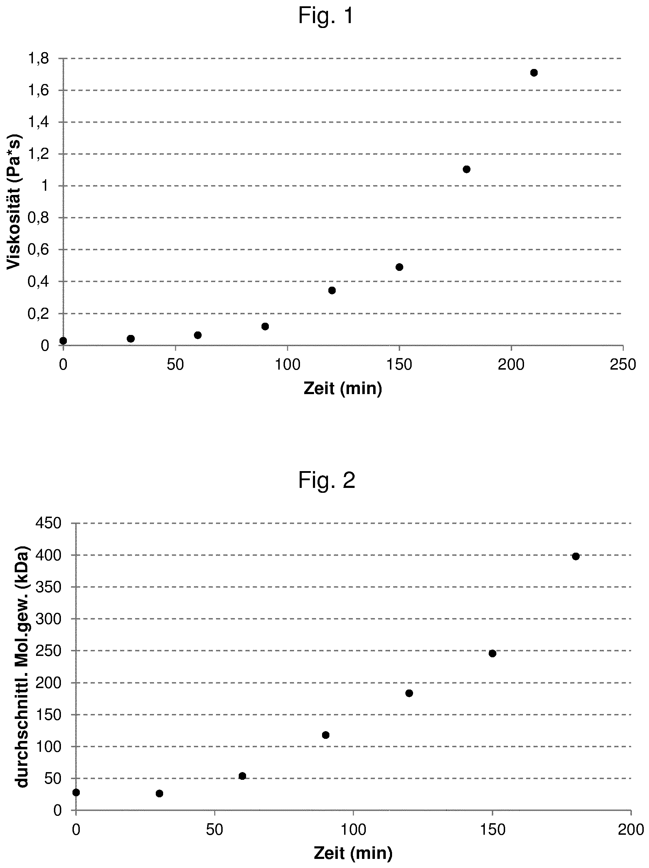

Unter Rühren mit einem Propellerrührer (600 U/min) wird durch eine Sinterfritte reiner Sauerstoff in die Lösung eingeblasen. Die Rate der Sauerstoffzugabe beträgt etwa 200 mL/min. Alle 30 Minuten wird eine Probe aus dem Reaktionsgemisch entnommen, von welcher die Viskosität und das durchschnittliche Molekulargewicht bestimmt werden. Der Verlauf der Viskosität in Abhängigkeit von der Reaktionsdauer ist in Fig. 1 gezeigt. Der Verlauf des durchschnittlichen Molekulargewichts in Abhängigkeit von der Reaktionsdauer ist in Fig. 2 gezeigt.

Aus Fig. 1 und Fig. 2 ist deutlich erkennbar, dass sowohl die Viskosität der Lösung als auch das Molekulargewicht der Lignosulfonat-Polymere mit der Reaktionsdauer zunehmen. Dies ist ein Indikator für die erfolgreiche Polymerisation der Lignosulfonat-Precursor.

Nach einer Reaktionszeit von 250 min werden die Rührung und die Zugabe von Sauerstoff beendet. Wird die Mischung stehen gelassen, bildet sich ein gelartiger Feststoff, welcher Lignosulfonat-Polymer und Wasser umfasst. Dieser gelartige Feststoff wird auch als Hydrogel bezeichnet.

Das erhaltene Hydrogel wird in Stücke von etwa 20 g aufgeteilt und bei 80°C für 12 Stunden in einem Trockenschrank getrocknet. Die Gewichtsreduktion während des Trocknens beträgt etwa 90 Gew.-%, woraus geschlossen werden kann, dass etwa 90 Gew.-% des Hydrogels Wasser sind. Nach dem vollständigen Trocknen wird wieder Wasser zum Polymer zugegeben, wobei sich zeigt, dass das Polymer rasch aufquillt und Wasser aufnimmt. Es kann nicht beobachtet werden, dass sich Teile des Polymers im Wasser lösen, das erhaltene Lignosulfonat-Polymer ist also wasserunlöslich.

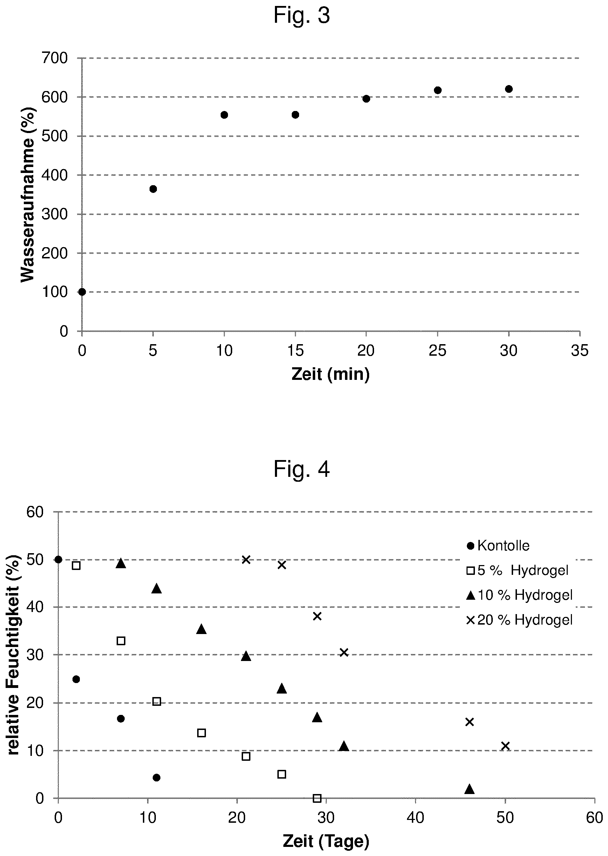

Die Wasseraufnahmefähigkeit des getrockneten Polymers wird durch Aufquellen der wie oben hergestellten Polymerstücke getestet. Der Verlauf der Wasseraufnahme ist in Fig. 3 gezeigt, wobei ersichtlich ist, dass die Wasseraufnahmemenge bereits nach einer Quelldauer von 20 min im Wesentlichen ein Plateau erreicht, welches bei etwa 600% der ursprünglichen Masse des trockenen Polymers liegt.

### Beispiel 2 - Lignosulfonat-Polymer als Wasserspeicher

In diesem Beispiel wird das im Rahmen des ersten Ausführungsbeispiels erhaltene Lignosulfonat-Polymer als Wasserspeicher in einem Wachstumssubstrat für Pflanzen eingesetzt. Das wieder aufgequollene Polymer aus Beispiel 1 wird in einem Anteil von etwa 5, 10 und 20 Gew.-%, mit einer Sand-Erde-Mischung vermischt. Zusätzlich wird eine Sand-Erde-Mischung ohne Zugabe von Polymer als Kontrolle vorbereitet.

Jeweils 200 g der so vorbereiteten Wachstumssubstrate werden in separate Töpfe gefüllt, wobei von jedem Substrat (Probe und Kontrolle) drei Töpfe vorbereitet werden. Die Substrate werden für 20 Stunden mit Wasser getränkt. Nach dieser Zeit wird überschüssiges Wasser abgegossen und jeweils drei Bohnensamen werden in jeden Topf ausgesät.

Von diesem Zeitpunkt an wird keine Bewässerung mehr vorgenommen, alle Töpfe werden bei gleicher Luftfeuchtigkeit, Temperatur und Beleuchtungsstärke belassen. Nach etwa 10 Tagen setzt die Keimung ein, wobei eine Keimungsrate von etwa 94% (17 von 18 Samen) beobachtet wird. Neben der Beobachtung des Wachstums der Keimlinge wird die relative Feuchtigkeit des Wachstumssubstrats in regelmäßigen Abständen gemessen. Der Verlauf der relativen Feuchtigkeit des Substrats (Mittelwert von je drei Töpfen die demselben Substrat) über die Zeit ist in Fig. 3 dargestellt. Während die relative Feuchtigkeit des Kontroll-Substrats bereits nach 11 Tagen nahe an 0% liegt, dauert die Austrocknung für die Hydrogel-enthaltenden Wachstumssubstrate länger. Beim Wachstumssubstrat mit 20 Gew.-% Hydrogel beträgt die relative Feuchtigkeit auch nach 50 Tagen noch etwa 10%.

Die in Fig. 3 gezeigte Beobachtung deckt sich mit dem Zustand der Keimlinge: Während bereits nach 15 Tagen ein deutliches Welken der Pflanzen in den Kontroll-Substraten einsetzt, zeigen die Pflanzen in den Hydrogel-enthaltenden Substraten über die gesamte Versuchsdauer von 50 Tagen keine sichtbaren Zeichen von Wassermangel.

### Beispiel 3 - Herstellung von Lignosulfonat-Polymer-Materialien

Für dieses Ausführungsbeispiel wird die Lignosulfonat-Polymerisation gemäß dem im ersten Ausführungsbeispiel beschriebenen erfindungsgemäßen Verfahren durchgeführt. Vor Abschluss der Polymerisation werden unterschiedliche Gewichtsanteile an Sorbitol oder Glycerin als Weichmacher zur der Reaktionsmischung gegeben und die Mischung wird homogenisiert. Es werden Mischungen mit 25 Gew.-%, 28 Gew.-%, 33 Gew.-%, 40 Gew.-% und 50 Gew.-% Sorbitol bzw. Glycerin hergestellt.

Nach Beenden der Sauerstoffzugabe und des Rührens wird die zähflüssige Reaktionsmischung auf eine Gusshöhe von etwa 1 mm, 2 mm und 4 mm in Petrischalen aus Kunststoff gegossen. Die gegossenen Schichten des Materials werden etwa 30 min bei 80°C trocknen gelassen. Die mechanischen Eigenschaften des erfindungsgemäßen Materials werden auf Basis der so erhaltenen Schichten getestet.

Nach dem Trocknen haben die Schichten oder Filme Dicken von etwa 0,10 mm bis 0,40 mm.

An den Materialien werden Versuche über die mechanische Beanspruchbarkeit durchgeführt.

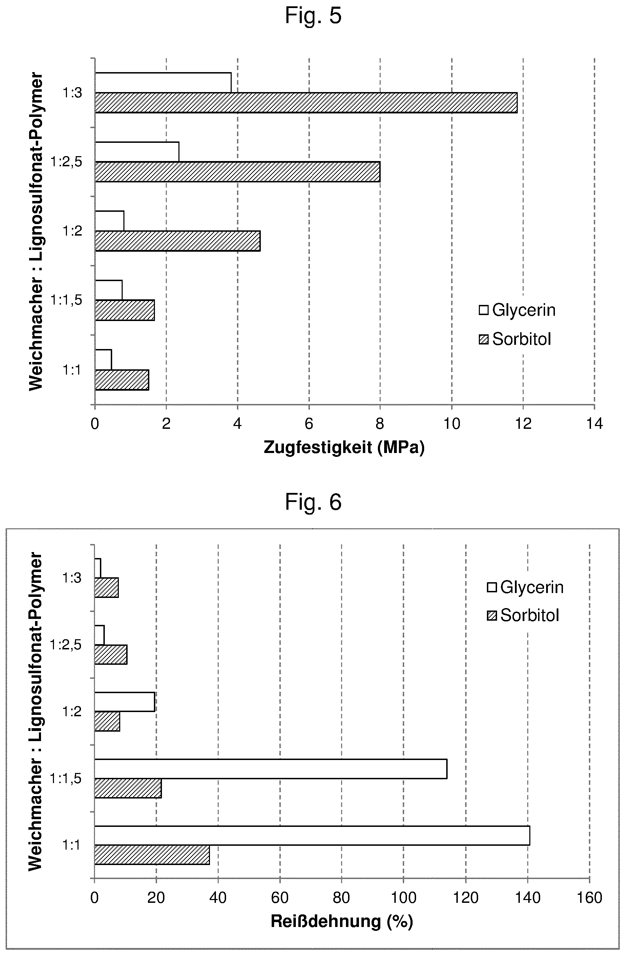

Fig. 5 zeigt die Zugfestigkeit der erhaltenen Materialien, Fig. 6 zeigt die Reißdehnung.

Es ist erkennbar, dass bei höherem Gehalt an Weichmacher eine niedrigere Zugfestigkeit erhalten wird. Bei Sorbitol ist die Zugfestigkeit höher als bei dem jeweils gleichen Gehalt an Glycerin.

Im Wesentlichen umgekehrt zur Zugfestigkeit verhält sich die Reißdehnung, welche in Fig. 6 dargestellt ist. Proben mit höherem Gehalt an Weichmacher zeigen größere Dehnung. Proben mit Glycerin zeigen größere Dehnung als Proben mit Sorbitol mit den jeweils gleichen Gehalten.

## Patentansprüche

1. **Verfahren** zur Herstellung eines wasserunlöslichen Lignosulfonat-Polymers aus einer Lignosulfonat-Precursor enthaltenden Lösung, nämlich aus Rohlauge, wobei der Lignosulfonat-Precursor eine Mischung aus wasserlöslichen Lignosulfonat-Oligomeren ist, und wobei das Verfahren die folgenden Schritte umfasst:
a. Abtrennen der Lignosulfonat-Oligomere und Bereitstellen einer Lösung mit wenigstens 5 Gew.-%, vorzugsweise wenigstens 10 Gew.-%, Lignosulfonat-Oligomeren, umfassend das Filtrieren von Bestandteilen mit einem Partikeldurchmesser von kleiner als 1 µm, insbesondere von kleiner als 100 nm, und das Abscheiden von niedermolekularen Bestandteilen mit einem Molekulargewicht von kleiner als 500 Da,
b. Zugabe eines Enzyms und Begasen der in Schritt (a) bereitgestellten Lignosulfonat-Oligomer-Lösung mit Sauerstoff zur Bildung von polymerisierbaren Lignosulfonat-Radikalen, wobei das Enzym dazu eingerichtet ist, die Bildung von Sauerstoff-Radikalen an phenolischen Hydroxylgruppen zu katalysieren, wobei das Enzym eine Laccase, eine Peroxidase oder eine Mischung aus mehreren dieser Enzyme ist, wobei Sauerstoff als Gas mit einer Beimischung von höchstens 10 Vol.-%, vorzugsweise höchstens 5 Vol.-%, an anderen Gasen zugegeben wird, und
c. Polymerisation der Lignosulfonat-Oligomere zu einem wasserunlöslichen Lignosulfonat-Polymer.

2. Verfahren nach Anspruch 1, **dadurch gekennzeichnet, dass** die Lignosulfonat-Oligomere ein Molekulargewicht von über 500 Da, bevorzugt von über 5000 Da, aufweisen.

3. Verfahren nach Anspruch 1 oder 2, **dadurch gekennzeichnet, dass** die Lösung in Schritt b) eine wässrige Lösung ist, wobei durch eine Sinterfritte Sauerstoff in die Lösung eingeblasen wird.

4. Verfahren nach einem der Ansprüche 1 bis 3, **dadurch gekennzeichnet, dass** der pH-Wert der Lösung in Schritt b) zwischen 5 und 9, bevorzugt zwischen 6 und 8, beträgt.

5. Verfahren nach einem der Ansprüche 1 bis 4, **dadurch gekennzeichnet, dass** während oder nach der Polymerisation wenigstens ein Zusatzstoff zugegeben wird, wobei der Zusatzstoff vorzugsweise ausgewählt ist aus Elastomeren, Weichmachern, Stabilisatoren und Polymeren.

6. Verfahren nach einem der Ansprüche 1 bis 5, **dadurch gekennzeichnet, dass** das Verfahren zusätzlich folgenden Schritt umfasst:
- Aufschäumen der Lignosulfonat-Oligomer-Lösung während der Polymerisation zur Bildung eines Lignosulfonat-Polymer-Schaumes.

7. Verfahren nach einem der Ansprüche 1 bis 5, **dadurch gekennzeichnet, dass** das Verfahren zusätzlich folgenden Schritt umfasst:
- Ausgießen der Lignosulfonat-Oligomer-Lösung während der Polymerisation der Lignosulfonat-Oligomere zur Bildung eines Lignosulfonat-PolymerMaterials, insbesondere eines Lignosulfonat-Biokunststoffs.

8. Verfahren nach einem der Ansprüche 1 bis 7, **dadurch gekennzeichnet, dass** die Aktivität des Enzyms in Schritt b) zwischen 10 nkat/mL und 500 nkat/mL, insbesondere zwischen 50 nkat/mL und 200 nkat/mL beträgt.

9. Verfahren nach einem der Ansprüche 1 bis 8, ferner umfassend den folgenden Schritt:
- Bilden eines Wachstumssubstrats für Pflanzen, das das wasserunlösliche Lignosulfonat-Polymer in einer Menge zwischen 0,1 Gew.-% und 5 Gew.-%, vorzugsweise zwischen 1 Gew.-% und 3 Gew.-%, enthält.

## Claims

1. A method for preparing a water-insoluble lignosulfonate polymer from a solution containing lignosulfonate precursors, namely from brown liquor, wherein the lignosulfonate precursor is a mixture of water-soluble lignosulfonate oligomers, the method comprising the following steps:
a. separating the lignosulfonate oligomers and providing a solution with at least 5% by weight, preferably at least 10% by weight, of lignosulfonate oligomers, comprising filtering out components having a particle diameter of less than 1 µm, in particular of less than 100 nm, and removing low-molecular components with a molecular weight of less than 500 Da,
b. adding an enzyme and gassing the lignosulfonate oligomer solution provided in step (a) with oxygen to form polymerizable lignosulfonate radicals, the enzyme being adapted to catalyze the formation of oxygen radicals at phenolic hydroxyl groups, the enzyme being a laccase, a peroxidase, or a mixture of several of these enzymes, oxygen being added as a gas with an admixture of not more than 10% by volume, preferably not more than 5 % by volume, of other gases, and
c. polymerizing the lignosulfonate oligomers to form a water-insoluble lignosulfonate polymer.

2. The method according to claim 1, **characterized in that** the lignosulfonate oligomers have a molecular weight of more than 500 Da, preferably of more than 5000 Da.

3. The method according to claim 1 or 2, **characterized in that** the solution of step b) is an aqueous solution, wherein oxygen is injected into the solution by a sinter frit.

4. The method according to one of claims 1 to 3, **characterized in that** the pH of the solution in step b) is between 5 and 9, preferably between 6 and 8.

5. The method according to one of claims 1 to 4, **characterized in that** at least one additive is added during or after the polymerization, wherein said additive is preferably selected from elastomers, plasticizers, stabilizers and polymers.

6. The method according to one of claims 1 to 5, **characterized in that** the method additionally comprises the following step:
- foaming the lignosulfonate oligomer solution during polymerization to form a lignosulfonate polymer foam.

7. The method according to one of claims 1 to 5, **characterized in that** the method additionally comprises the following step:
- pouring the lignosulfonate oligomer solution during the polymerization of the lignosulfonate oligomer to form a lignosulfonate polymer material, in particular a lignosulfonate bioplastic.

8. The method according to one of claims 1 to 7, **characterized in that** the activity of the enzyme in step b) is between 10 nkat/mL and 500 nkat/mL, in particular between 50 nkat/mL and 200 nkat/mL.

9. The method according to one of claims 1 to 8, further comprising the following step:
- forming a growth substrate for plants, which contains the water-insoluble lignosulfonate polymer in an amount between 0.1% by weight and 5% by weight, preferably between 1% by weight and 3% by weight.

## Revendications

1. **Procédé** de préparation d'un polymère de lignosulfonate insoluble dans l'eau à partir d'une solution contenant un précurseur de lignosulfonate, à savoir une liqueur brute, dans lequel le précurseur de lignosulfonate est un mélange d'oligomères de lignosulfonate solubles dans l'eau, et dans lequel le procédé comprend les étapes suivantes :
a. Séparation des oligomères de lignosulfonate et fourniture d'une solution contenant au moins 5 % en poids, de préférence au moins 10 % en poids, d'oligomères de lignosulfonate, comprenant la filtration des composants ayant un diamètre de particule inférieur à 1 µm, en particulier inférieur à 100 nm, et la séparation des composants de faible poids moléculaire ayant un poids moléculaire inférieur à 500 Da,
b. l'ajout d'une enzyme et la gazéification de la solution d'oligomères de lignosulfonate fournie dans l'étape (a) avec de l'oxygène pour former des radicaux de lignosulfonate polymérisables, l'enzyme étant adaptée pour catalyser la formation de radicaux oxygène sur les groupes hydroxyle phénoliques, l'enzyme étant une laccase, une peroxydase ou un mélange de plusieurs de ces enzymes, l'oxygène étant présent sous forme de gaz avec un mélange d'au plus 10 % en volume d'oxygène, de préférence au plus 5 % en volume, d'autres gaz, et
c. la polymérisation des oligomères de lignosulfonate en un polymère de lignosulfonate insoluble dans l'eau.

2. Procédé selon la revendication 1, **caractérisé en ce que** les oligomères de lignosulfonate présentent un poids moléculaire supérieur à 500 Da, de préférence supérieur à 5 000 Da.

3. Procédé selon la revendication 1 ou 2, **caractérisé en ce que** la solution à l'étape b) est une solution aqueuse, de l'oxygène étant insufflé dans la solution par une fritte de frittage.

4. Procédé selon l'une des revendications 1 à 3, **caractérisé en ce que** le pH de la solution à l'étape b) est compris entre 5 et 9, de préférence entre 6 et 8.

5. Procédé selon l'une des revendications 1 à 4, **caractérisé en ce qu'**au moins un additif est ajouté pendant ou après la polymérisation, ledit additif étant de préférence choisi parmi les élastomères, les plastifiants, les stabilisants et les polymères.

6. Procédé selon l'une des revendications 1 à 5, **caractérisé en ce que** le procédé comprend en outre l'étape suivante :
- Faire mousser la solution d'oligomère de lignosulfonate pendant la polymérisation pour former une mousse de polymère de lignosulfonate.

7. Procédé selon l'une des revendications 1 à 5, **caractérisé en ce que** le procédé comprend en outre l'étape suivante :
- Verser la solution d'oligomères de lignosulfonate pendant la polymérisation des oligomères de lignosulfonate pour former un matériau polymère de lignosulfonate, en particulier un bioplastique de lignosulfonate.

8. Procédé selon l'une des revendications 1 à 7, **caractérisé en ce que** l'activité de l'enzyme à l'étape b) est comprise entre 10 nkat/mL et 500 nkat/mL, notamment entre 50 nkat/mL et 200 nkat/mL.

9. Procédé selon l'une quelconque des revendications 1 à 8, comprenant en outre l'étape suivante :
- former un substrat de croissance pour plantes contenant le polymère lignosulfonate insoluble dans l'eau en une quantité comprise entre 0,1 % et 5 % en poids, de préférence entre 1 % et 3 % en poids.
